# FASCICULE DE BREVET EUROPEEN

(11) **EP 1 076 553 B1**
(45) Date de publication et mention de la délivrance du brevet: **09.08.2006**
(21) Numéro de dépôt: 99918205.8
(22) Date de dépôt: 12.05.1999
(51) Int. Cl.: A61K 8/44, A61K 8/96, A61Q 17/00, A61Q 19/00, A61K 35/08

(54) **COMPOSITIONS PHARMACEUTIQUES ET/OU COSMETIQUES CONTENANT DE L'EAU DE MER**
PHARMAZEUTISCHE UND/ODER KOSMETISCHE ZUSAMMENSETZUNGEN ENTHALTEND MEERWASSER
PHARMACEUTICAL, HYGIENIC AND/OR COSMETIC COMPOSITIONS CONTAINING SEA WATER AND USES

(30) Priorité: 14.05.1998 FR 9806119
(43) Date de publication de la demande: 21.02.2001
(60) Demande divisionnaire: 04291555.3
(73) Titulaire: Sephra S.A.R.L., 75016 Paris (FR)
(72) Inventeur: JOLY, Francine, F-75016 Paris (FR); BEAUVAIS, Francis, F-92310 Sèvres (FR)
(74) Mandataire: Burtin, Jean-François
(86) Numéro de dépôt international: PCT/IB1999/000862
(87) Numéro de publication internationale: WO 1999/058095

(56) Documents cités:
- EP-A- 0 908 184
- WO-A-97/05862
- CH-A- 500 711
- DE-A- 4 341 000
- FR-A- 1 248 181
- FR-A- 2 142 589
- FR-A- 2 242 971
- FR-A- 2 590 270
- FR-A- 2 590 273
- FR-A- 2 711 990
- FR-A- 2 740 339
- FR-A- 2 741 535
- STN, Serveur de Bases de Données, Karlsruhe, DE, Fichier Chemical Abstracts, Vol 130, AN= 242149 résumé XP002117253 & JP 11 060468 A (KANEBO)
- PATENT ABSTRACTS OF JAPAN vol. 010, no. 120 (C-343) & JP 60 246360 A (AJINOMOTO KK)

## Description

La présente invention se rapporte au domaine de la chimie thérapeutique et plus particulièrement à celui de la pharmacie, de l'hygiène et/ou de la cosmétologie.

La présente invention a pour objet une composition pharmaceutique, hygiénique et/ou cosmétique contenant de l'eau de mer et un aminoacide basique ou un de ses sels ou un de ses esters. Les principaux aminoacides basiques sont l'arginine, la lysine, la citrulline ou l'ornithine.

Les compositions pharmaceutiques, hygiéniques et/ou cosmétiques conformes à l'invention présentent une action inhibitrice de l'activation des mastocytes induite par des neuropeptides. En particulier, les compositions selon l'invention présentent une action inhibitrice sur la dégranulation des mastocytes induite par la substance P. En conséquence, ces compositions manifestent des effets anti-inflammatoires et/ou anti-allergiques et peuvent être utilisées pour prévenir et/ou traiter des affections liées à une libération d'histamine.

La peau et les muqueuses sont le siège d'agressions multiples auxquelles elles répondent par une réaction inflammatoire. Ainsi, les stimuli nociceptifs (température, stimuli mécaniques, irritants chimiques, allergènes, UV...) provoquent la libération de neuromédiateurs, en particulier de la substance P. Ces substances sont capables d'induire une réaction inflammatoire qui est alors dite « neurogène ». Il est important de noter que l'inflammation neurogène est en fait une composante de toute inflammation quelle qu'en soit la cause (Ratzlaff et al, 1992, J. Neuroimmunol. 41:89-96). Par conséquent toute substance capable d'interférer avec les effets de la substance P est susceptible d'avoir un effet anti-inflammatoire, anti-allergique, insensibilisant et antalgique.
Les neurokinines constituent une famille de peptides qui sont libérés par les nerfs sensitifs. Cette famille comprend la substance P, les neurokinines A et B. Les neurokinines ainsi que le CGRP (calcitonin gene related peptide) et le VIP (vasoactive intestinal peptide) sont des médiateurs du système nerveux périphérique NANC (non adrénergique, non cholinergique). Tous ces peptides peuvent être libérés par les fibres nerveuses sensitives (fibres C) qui innervent la peau. Ce sont principalement des médiateurs inflammatoires. Libérés dans la peau, les neurokinines, et notamment la bradykinine, induisent démangeaisons, rougeurs, oedème...

Ces symptômes sont liés en grande partie à la libération d'histamine par la substance P à partir des mastocytes cutanés.
La substance P a un effet stimulant sur la prolifération des lymphocytes, la synthèse d'immunoglobulines, la dégranulation des mastocytes, la phagocytose des macrophages, le chimiotactisme et la libération de médiateurs par les neutrophiles. La substance P est donc un facteur très important de l'inflammation neurogène. L'injection intradermique de substance P chez l'homme ou l'animal (souris, cobaye) provoque un érythème en quelques minutes. Au point d'injection, l'étude histologique révèle une augmentation importante du nombre de neutrophiles et d'éosinophiles intra- et périvasculaires. Le mécanisme de cette accumulation de cellules inflammatoires dans la peau semble impliquer deux voies (Smith et al, 1993, J. Immunol. 151:3274-3282). D'une part, la substance P induit la dégranulation des mastocytes cutanés provoquant ainsi la libération de médiateurs de l'inflammation tels que l'histamine et des médiateurs chimiotactiques pour les polynucléaires (LTB4, Paf-acéther). D'autre part, la substance P augmente l'expression de molécules d'adhésion par les cellules endothéliales microvasculaires du derme, et induit la libération de cytokines pro-inflammatoires par les mastocytes. Une grande partie des effets inflammatoires de la substance P au niveau de la peau sont donc liés à la dégranulation des mastocytes cutanés (mastocytes de type séreux). Des études histologiques et ultrastructurales ont montré que les fibres C étaient en contact étroit avec les mastocytes cutanés. Les libérations de substance P et d'histamine s'amplifient donc mutuellement dans une boucle d'auto-entretien.

Selon la présente invention, le demandeur a découvert, de façon surprenante, que l'eau de mer prise isolément, ainsi que l'aminoacide basique pris isolément, présentent chacun une action inhibitrice de l'activation des mastocytes induite par des neuropeptides, et, en particulier une action inhibitrice sur la dégranulation des mastocytes induite par la substance P. En conséquence, l'eau de mer, tout comme l'aminoacide basique, sont des inhibiteurs de la libération d'histamine induite par la substance P. De plus, le demandeur a découvert, d'une manière tout à fait inattendue que l'association de ces deux constituants manifeste un effet synergique net sur l'inhibition de l'activation des mastocytes induite par des neuropeptides, et, en particulier sur l'inhibition de la dégranulation des mastocytes induite par la substance P.

Ainsi, les compositions conformes à l'invention, et notamment celles contenant l'association eau de mer-aminoacide basique constituent, grâce à leur action inhibitrice sur la libération d'histamine, un progrès technique important pour le traitement des manifestations allergiques et/ou inflammatoires.
En effet, l'allergie et l'inflammation (notamment cutanée), posent encore aujourd'hui de nombreux problèmes aux thérapeutes qui ne disposent que d'un nombre limité de substances actives. En outre, certaines de ces substances, comme par exemple les corticoïdes, peuvent présenter des effets secondaires souvent pénalisants (atrophies, vieillissement de la peau, infections mycotiques ou bactériennes etc.).

La présente invention a plus particulièrement pour objet une composition pharmaceutique, hygiénique et/ou cosmétique destinée notamment à inhiber la dégranulation des mastocytes caractérisée en ce qu'elle contient de l'eau de mer et un aminoacide basique ou l'un de ses sels ou esters, ou un extrait végétal et/ou animal en contenant, en association ou en mélange avec un véhicule ou un excipient inerte, non toxique, approprié pour l'application envisagée.

Elle a aussi pour objet l'utilisation de l'eau de mer en vue de la réalisation d'une composition pharmaceutique, hygiénique et/ou cosmétique destinée notamment à inhiber l'activation des mastocytes, en particulier la dégranulation.

Elle a encore pour objet l'utilisation d'un aminoacide basique, et notamment de l'arginine ou de l'un de ses sels ou esters, ou d'un extrait végétal et/ou animal en contenant, en vue de la réalisation d'une composition pharmaceutique, hygiénique et/ou cosmétique destinée notamment à inhiber l'activation des mastocytes, en particulier la dégranulation.

Les aminoacides basiques ont de nombreuses propriétés pharmacologiques. Le principal représentant est l'arginine, qui est un acide aminé naturel à reste guanidine. Il est, sous l'effet de la NO-synthase, à l'origine de la formation du monoxyde d'azote. La L-arginine est connue pour ses propriétés bioénergétiques et antiasthéniques, comme stimulant la biosynthèse de l'hormone de croissance, contre la sénescence du cristallin, ainsi que pour lutter contre l'hyperammoniémie et ses conséquences.
Selon l'invention, l'arginine utilisée peut être d'origine naturelle ou synthétique.
Un exemple d'extrait végétal et/ou animal contenant un aminoacide basique est un extrait d'algue, un extrait de boue marine, thermale ou lacustre, un extrait de bactérie. Ces extraits végétaux et/ou animaux proviennent notamment de péloïdes. Ainsi, des compositions contenant de l'eau de mer associée par exemple à des extraits de boues marines, sont, dans la mesure où ces extraits contiennent un aminoacide basique, notamment de l'arginine, conformes à l'invention.
L'arginine, quand elle est obtenue par voie naturelle, est sous forme lévogyre (L-arginine).
L'arginine utilisée de préférence dans la présente invention, est la L-arginine.
L'arginine peut également être obtenue par voie synthétique, sous forme racémique. On pourra, selon l'invention, utiliser la DL-arginine ou bien encore utiliser de la D-arginine.
L'arginine peut être utilisée soit sous forme libre, soit sous forme de l'un de ses sels ou de ses esters pharmaceutiquement et/ou cosmétiquement acceptables. Parmi les sels d'arginine, on pourra citer le mono- ou dichlorhydrate, le mono- ou dibromhydrate, le sulfate, le glutamate, le pidolate ou le chlorhydrate. Parmi les esters on pourra citer l'ester méthylique ou l'ester éthylique de ceux-ci.

L'eau de mer utilisée dans la présente invention est prélevée notamment dans des mers ou océans, mais aussi dans des résurgences ou infiltrations où circule l'eau de mer. Elle peut être filtrée et stérilisée par filtration stérilisante complémentaire.
Les mers et océans sont par exemple l'Atlantique ou la Manche. L'eau de mer est filtrée sur un filtre assez large afin d'éliminer les particules solides en suspension, et elle est stérilisée par passage sur une membrane stérilisante.
L'eau de mer ainsi filtrée et stérilisée peut ensuite, selon les usages envisagés, être amenée à l'isotonie par dilution ou par désionisation.
L'eau de mer peut être désionisée par adsorption sélective du sodium et/ou remplacement du sodium par un autre ion métallique comme le calcium ou le magnésium. On peut ainsi renforcer la teneur de l'eau de mer en sels de calcium et/ou de magnésium tout en diminuant la teneur en sels de sodium ou de potassium. De la même façon on peut modifier la teneur en bromates et/ou en bromures.

Dans les compositions conformes à l'invention, contenant l'association eau de mer-aminoacide basique, la quantité d'eau de mer représente de 30 % à 99 % du poids total de la composition. Plus particulièrement, la quantité d'eau de mer représente de 60 % à 95 % du poids total de la composition. La quantité d'aminoacide basique, dans les compositions contenant l'association eau de mer-aminoacide, représente de 0,0001 % à 10 % du poids total de la composition. Plus particulièrement, la quantité d'aminoacide basique représente de 0,0005 % à 2 % du poids total de la composition.

Cependant, lorsque l'eau de mer est utilisée seule, ou, inversement lorsque l'aminoacide basique est utilisé seul, en vue de la réalisation d'une composition pharmaceutique, diététique et/ou cosmétique destinée notamment à inhiber la dégranulation des mastocytes, les pourcentages seront différents.
Les modalités données ci-dessous se réfèrent plus particulièrement à la composition telle que définie précédemment, contenant l'eau de mer et un aminoacide basique comme l'arginine.
Cependant, ces modalités sont également applicables lorsque l'eau de mer est utilisée seule, ou, inversement lorsque l'aminoacide basique est utilisé seul, en vue de la réalisation d'une composition pharmaceutique, hygiénique et/ou cosmétique destinée notamment à inhiber la dégranulation des mastocytes.

On peut, en outre, associer à la composition selon l'invention des agents actifs supplémentaires destinés notamment à la prévention et/ou au traitement d'affections inflammatoires et/ou allergiques. Ainsi, la composition est encore caractérisée en ce qu'elle contient au moins un agent choisi parmi les agents antibactériens, antiparasitaires, antifongiques, antiprurigineux, anti-radicaux libres, anesthésiques, antiviraux, antipelliculaires, anti-acnéiques, des antiséborrhéiques, des vitamines et/ou des agents cicatrisants, et/ou prévenant ou traitant le vieillissement (de la peau, des gencives, ...), les agents tenseurs.

La composition conforme à l'invention peut en outre renfermer un agent de régulation du pH. Le pH de la composition conforme à l'invention est réglé dans une zone s'étendant de 5,5 à 9, de préférence de 6 à 8. L'agent régulateur du pH est par exemple un tampon comme un phosphate de métal alcalin ou un mélange de phosphates mono- et di-alcalins.

Les compositions conformes à l'invention sont particulièrement utilisées pour prévenir et/ou traiter les affections liées à une libération de médiateurs de l'inflammation et/ou de l'allergie tels que l'histamine ou les cytokines.
Des exemples de telles affections sont notamment les manifestations allergiques et/ou inflammatoires, quelle qu'en soit l'origine et le point d'application, notamment la peau, les yeux, les bronches et le nez.
Ainsi, lesdites compositions sont destinées notamment à la prévention et/ou au traitement de l'urticaire, de l'eczéma, du psoriasis, des rougeurs ou irritations cutanées, des prurits, des dartres, des érythèmes (en particulier solaires), des piqûres d'insectes, des brûlures, des conjonctivites allergiques, de l'asthme bronchique allergique ou de l'effort, du rhume des foins, des rhinites et trachéites spasmodiques. Elles peuvent aussi être utilisées en ORL chez les adultes mais aussi chez les nourrissons et les jeunes enfants (décongestion du nez ou lavage des muqueuses), lorsque ceux-ci présentent des surinfections pharyngées, ou sont enrhumés, ou encore, lorsque la muqueuse nasale est congestionnée. Lesdites compositions pourront encore être utilisées pour traiter les pathologies veineuses comme par exemple les thrombophlébites, les troubles liés à l'insuffisance veineuse lymphatique (cellulite, jambes lourdes...)...
Les compositions conformes à l'invention peuvent également être destinées aux lignes de produits hypoallergéniques et/ou pour peaux allergiques, pour les peaux sensibles (irritables, réactives, intolérantes), pour l'usage bucco-dentaire et pour la cicatrisation des plaies et des traumatismes.
Les compositions selon l'invention trouvent aussi un emploi dans la prévention ou le traitement du vieillissement cutané.

Selon la voie d'administration et l'usage envisagé, la composition pharmaceutique, hygiénique et/ou cosmétique se présentera sous une des quelconques formes galéniques normalement utilisées. La composition pourra se présenter sous forme solide ou liquide ou lyophilisée. La forme solide sera par exemple en comprimés, en capsules, en gélules, en pilules, en crème, en gel, en pommade, en émulsion solide. La forme liquide sera par exemple une solution, un soluté, une suspension, un collyre, un sérum, une lotion, un lait, une émulsion huile dans l'eau ou eau dans l'huile. On pourra administrer les compositions selon l'invention sous forme de patches.
La voie d'administration envisagée pourra être la voie cutanée, orale, percutanée, parentérale, nasale, oculaire, buccale, gingivale, bronchique, vaginale, rectale... De même, les excipients utilisés sont ceux qui sont normalement appropriés selon la voie d'administration et l'usage envisagé.

La composition selon l'invention est de préférence destinée à être appliquée sur la peau (sur toute zone cutanée du corps) et les muqueuses, en particulier nasales ou oculaires.
Ainsi, pour une application à visée thérapeutique concernant les yeux, les compositions de l'invention peuvent se présenter sous forme de collyre, de pommade ou de solution de lavage oculaire.

Parmi les aminoacides basiques de l'invention, on pourra citer plus particulièrement :
- ceux à fonction guanidine, comme l'arginine, l'homoarginine ;
- ceux à fonction amine, comme la lysine, l'acide diaminopimelique ou l'acide diaminovalérique ;
- ceux à fonction ammonium quaternaire, comme la carnitine, l'homarine ;
- ceux substitués par un méthyle, comme l'α-méthyl m.tyrosine ou l'acide N-méthylaspartique ;
- ceux substitués par un groupement carboxamide, comme l'ornithine ;
- ceux substitués par un groupe cyano :
- ceux substitués par un groupe méthylamino, comme la sarcosine ;
- ceux substitués par un groupe phosphonique.

### PARTIE EXPERIMENTALE

### Etude de l'action de l'eau de mer seule, de l'arginine seule, et, de la composition contenant à la fois l'eau de mer et l'arginine sur la libération d'histamine.

### I) Modèle in vitro

On étudie ici les effets inhibiteurs de l'eau de mer seule, de l'arginine seule, et, de l'eau de mer supplémentée en arginine, sur l'inflammation neurogène. Ainsi, on étudie in vitro, si l'eau de mer supplémentée en arginine inhibe la dégranulation des mastocytes péritonéaux de rat induite par la substance P, et, si ladite eau de mer supplémentée en L-arginine présente un effet synergique net par rapport à chacun des deux constituants pris isolément.
Les mastocytes péritonéaux de rat sont considérés en pharmacologie comme un modèle des mastocytes cutanés humains.

### a) Introduction

Il existe deux sous-populations mastocytaires distinctes : les mastocytes « muqueux » (appelés autrefois atypiques) présents classiquement dans les muqueuses, et, les mastocytes séreux (ou « mastocytes du tissu conjonctif ») présents dans la peau et la cavité péritonéale. Ces deux sous-classes sont distinctes par leur localisation tissulaire, leurs propriétés histologiques, immunologiques et fonctionnelles. Les mastocytes de la cavité péritonéale de rat sont un modèle pharmacologique classique des mastocytes cutanés humains.
L'activation des mastocytes peut non seulement faire intervenir la stimulation de leurs récepteurs spécifiques des IgE mais également leur réactivité à différents peptides. Ainsi, la voie d'activation peptidergique des mastocytes constitue la seconde voie d'activation physiologique de ces cellules, à côté de la voie dépendante des IgE (voie antigénique).

### b) Protocole expérimental

Les mastocytes péritonéaux de rat sont obtenus par lavage après injection d'une solution de Tyrode. Les mastocytes représentent 8 à 10 % des cellules péritonéales composées également de macrophages, lymphocytes et monocytes. Les mastocytes sont purifiés sur un coussin de métrizamide à 22,5 % puis remis en suspension dans une solution de Tyrode.

### Composition de la solution de Tyrode (en mM) :

- NaCl : 137
- KCl : 2,6
- glucose : 5,6
- HEPES : 4,2
- CaCl₂ : 0,3
- Sérum albumine bovine : 0,25 %

Les mastocytes sont préincubés (5 min; 37° C au bain-marie) avec un témoin (le tampon Tyrode), avec différentes concentrations d'eau de mer seule, d'arginine seule, ou d'eau de mer supplémentée en arginine, dans le tampon Tyrode.
Les mastocytes sont ensuite stimulés par la substance P (5 min; 37° C). De l'acide perchlorique (0,4N final) est ajouté sur les culots et les surnageants cellulaires puis l'histamine est mesurée par une méthode spectrofluorimétrique.

### c) Résultats

Les résultats sont donnés dans le tableau 1 ci-dessous :

**Tableau 1 :**

| Pourcentage d'histamine libérée | Arginine 0mM | Arginine 1mM | Arginine 3mM |
|---|---|---|---|
| 0% eau de mer | 55 ± 8 | 38 ± 6 | 19 ± 4 |
| 3%eaudemer | 35 ± 7 | 22 ± 5 | 6 ± 3 |
| 10% eau de mer | 6 ± 1 | 2 ± 1 | 1 ± 1 |
| 20% eau de mer | 1 ± 0 | 1 ± 0 | 1 ± 0 |

La colonne 1 du tableau 1 représente les résultats obtenus avec respectivement le témoin, i.e 0% eau de mer, 100% solution Tyrode, une concentration d'eau de mer à 3 % + 97% solution Tyrode, une concentration d'eau de mer à 10 % + 90% solution Tyrode, et, une concentration d'eau de mer à 20 % + 80% solution Tyrode. La colonne 2 représente les résultats obtenus avec respectivement les mêmes concentrations (eau de mer/solution Tyrode) que dans la colonne 1 avec en plus de l'arginine à une concentration de 1mM. De même, la colonne 3 représente les résultats obtenus avec respectivement les mêmes concentrations (eau de mer/solution Tyrode) que dans la colonne 1, avec en plus de l'arginine à une concentration de 3mM.

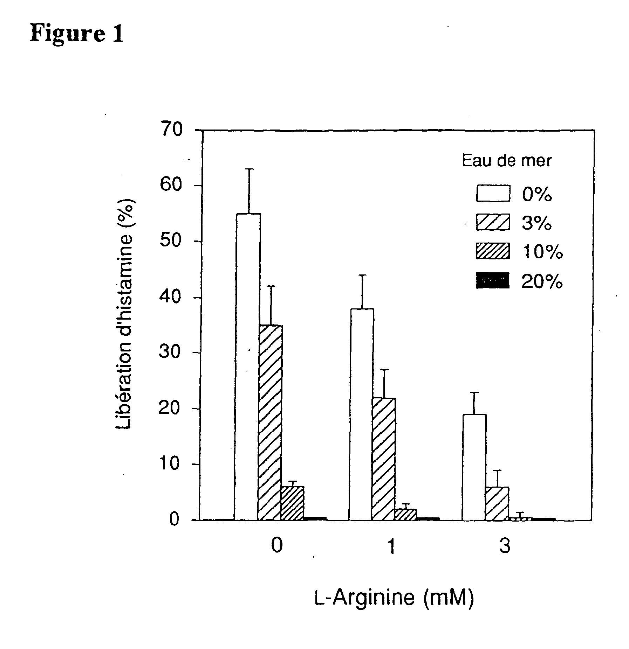

Les résultats du tableau 1 sont exprimés en pourcentages de libération d'histamine ± S.E.M.
n = 6 expériences
Le tableau 1 est représenté par la figure 1.

| Les symboles □, , 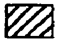 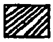 , ■, de la figure 1 représentent : | | |
|---|---|---|
| □ | 0 % eau de mer (100% Solution Tyrode) | (supplémenté éventuellement en L-arginine) |
| 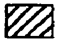 | 3 % eau de mer (97 % Solution Tyrode) | (") |
| 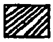 | 10 % eau de mer (90% Solution Tyrode) | (") |
| ■ | 20 % eau de mer (80% Solution Tyrode) | (") |

Le pourcentage de libération d'histamine induite par la stimulation peptidergique est de 55 ± 8 %. En présence d'eau de mer seule, la libération d'histamine diminue plus ou moins fortement en fonction de la concentration d'eau de mer dans la préparation. Ainsi, pour une concentration d'eau de mer de 3% dans la préparation, la libération d'histamine est de 35 ± 7 % ; pour une concentration d'eau de mer de 10%, la libération d'histamine est de 6 ± 1 % et, en présence d'une concentration d'eau de mer de 20%, la libération d'histamine n'est plus que de 1 ± 0 %.
Ainsi, même en présence d'un pourcentage faible d'eau de mer (3%), on peut déjà noter une inhibition de la dégranulation mastocytaire. L'inhibition de la libération d'histamine est d'autant plus importante que la concentration en eau de mer est élevée.

De même, en présence d'arginine seule, la libération d'histamine diminue également. Ainsi, en présence d'arginine seule à une concentration de 1 mM, la libération d'histamine est de 38 ± 6 %, et, en présence d'arginine seule à 3 mM, la libération d'histamine n'est plus que de 19 ± 4 %.

Ainsi, le traitement des mastocytes par l'eau de mer seule, ou, par l'arginine seule, induit une inhibition dose-dépendante de la dégranulation des masrtocytes induite par la substance P.

Le traitement des mastocytes par la composition selon l'invention, contenant de l'eau de mer et un aminoacide basique comme l'arginine, potentialise l'inhibition de la libération d'histamine par rapport à celle observée lorsque les cellules sont incubées uniquement en présence d'eau de mer ou d'arginine.

### d) Conclusion

Les effets inhibiteurs de l'eau de mer et d'un aminoacide basique comme l'arginine se potentialisent mutuellement. On constate expérimentalement une efficacité accrue de ladite association sur l'inhibition de la dégranulation des mastocytes par rapport à celle de l'eau de mer seule ou de l'arginine seule.

Le tableau II ci-après reprend les résultats précédents fournis au tableau I sur l'inhibition de la libération d'histamine par l'eau de mer après induction par la substance P.

Ces résultats sont complétés :
- par l'étude de l'inhibition de la dégranulation des basophiles humains provoquée par un sérum anti IgE ;
- par l'étude de la dégranulation des mastocytes intrapéritonéaux de rats après induction par le VIP ; l'effet inhibiteur est totalement sous la dépendance de la concentration en eau de mer ;
- par l'étude de la dégranulation des mastocytes péritonéaux de rats induite par le CGRP ; on obtient à une concentration de 10 % en eau de mer une inhibition déjà très importante ;
- par l'étude de la dégranulation des mastocytes péritonéaux de rats induite par la bradykinine. L'inhibition de la dégranulation est pratiquement totale pour une concentration d'eau de mer à 10 %.

**Tableau 2**

| **Résumé de l'inhibition de la libération d'histamine par l'eau de mer** | | |
|---|---|---|
| **Mastocytes péritonéaux du rat (SP 10µM)** | | |
| n=7 | **Moyenne** | **SEM** |
| SP | 55,5 % | 6,7 % |
| SP + EM 3 % | 34,7 % | 6,2 % |
| SP + EM 10 % | 6,8 % | 1,5 % |
| SP + EM 20 % | 1,4 % | 1,3 % |
| | | |

| **Basophiles humains (anti-IgE 1/1000)** | | |
|---|---|---|
| n=3 | **Moyenne** | **SEM** |
| a-IgE | 52 % | 9 % |
| a-IgE + eau de mer 3 % | 26 % | 2 % |
| a-IgE + eau de mer 10 % | 17 % | 2 % |
| a-IgE + eau de mer 20 % | 10 % | 1 % |
| | | |

| **Mastocytes péritonéaux de rats (VIP 3µM)** | | |
|---|---|---|
| n=4 | **Moyenne** | **SEM** |
| VIP | 61% | 3 % |
| VIP + eau de mer 3 % . | 44 % | 5 % |
| VIP + eau de mer 10 % | 14 % | 3 % |
| VIP + eau de mer 20 % | 4 % | 2 % |
| | | |

| **Mastocytes péritonéaux de rats (CGRP 30µM)** | **exp 1** | |
|---|---|---|
| n=1 | | |
| CGRP | 79 % | |
| CGRP + 10 % eau de mer | 44 % | |
| | | |

| **Mastocytes péritonéaux de rats (Bradykinine 30µM)** | **exp 1** | |
|---|---|---|
| n=1 | | |
| Bradykinine | 67 % | |
| Bradykinine + 10 % eau de mer | 7 % | |

| | | |
|---|---|---|
| SP : substance P | | |
| EM : eau de mer | | |
| VIP : Vasoactive Intestinal Peptide | | |

### II) Modèle in vivo

### 1. Effet de la composition contenant l'association eau de mer-arginine sur un modèle in vivo d'extravasement cutané.

### a) Introduction

L'injection sous cutanée de substance P chez le rat ou le cobaye produit une inflammation de la peau du dos se traduisant par une vasodilatation cutanée et un extravasement des protéines plasmatiques. Ce modèle d'inflammation neurogène permet de tester des substances à visée anti-allergiques et/ou anti-inflammatoires. L'extravasement plasmatique liée à l'inflammation neurogénique est mis en évidence par le bleu d'Evans.

### b) Protocole expérimental

Le dos de cobayes (mâles Hartley, 300 g) est rasé et une solution isotonique de bleu Evans est injectée dans la veine du pénis. Cent µl de substance P (0,62µmol) sont dilués dans du sérum physiologique en présence ou non d'une solution d'eau de mer à 10 % et d'arginine (1 µmol) et ont été injectés en différents sites sous cutanés. L'extravasement plasmatique est visualisé par le bleuissement des téguments.

### c) Résultats

Les résultats sont illustrés par le tableau 3. Les croix symbolisent l'intensité du bleuissement des téguments.

**Tableau 3 :**

| Stimulus | Intensité de la réaction colorée |
|---|---|
| Substance P (SP) | +++ |
| SP + eau de mer 10% | ++ |
| SP + eau de mer 10% + arginine | + |

### d) Conclusion

L'eau de mer seule à 10 % diminue l'extravasement induit par la substance P. Cet effet est potentialisé par l'arginine.

### 2. Essai « in vivo »

### Evaluation de l'effet anti-inflammatoire sur l'inflammation neurogénique induite par la stimulation électrique de la veine saphène chez le rat.

### a) Principe

Tout effet anti-inflammatoire neurogénique éventuel de l'eau de mer enrichie en arginine sur l'inflammation neurogénique induite par stimulation électrique de la veine saphène a été évalué chez le rat anesthésié. Le test consiste à induire une inflammation neurogénique par stimulation de la veine saphène, ce nerf innervant la zone cutanée de la patte arrière. Sa stimulation induit la libération, à partir des terminaisons nerveuses, de neuromédiateurs responsables de l'inflammation neurogénique tels que la substance P ou le CGRP. L'inflammation neurogénique est appréciée par la mesure de l'extravasement du Bleu Evans qui survient au cours des processus inflammatoires.

### b) Méthode expérimentale

Des rats Wistar mâles d'un poids moyen de 250 g ont été logés dans des cages de dimensions standard sous air conditionné.
Les animaux ont été répartis en quatre lots :
- le groupe 1 est un lot témoin recevant de l'eau bidistillée ;
- le groupe 2 est un lot témoin pour la méthode qui reçoit une substance témoin (Spantide II) qui est un antagoniste de la substance P à la dose de 30 nmol/animal ;
- et le groupe 3 est un lot qui reçoit l'eau de mer enrichie avec un aminoacide basique.

La veille de l'essai (jour 4) les animaux ont été traités avec de la guanethidine (20 mg/Kg s.c. à la dose de 1 mg/Kg pour éviter toute interaction avec les catécholamines.

Le jour de l'essai (jour 5), les animaux ont reçu selon le plan de randomisation le produit prévu puis ils ont été anesthésiés avec du pentobarbital (60 mg/Kg IP à la dose de 1 mg/Kg). Environ 15 minutes après la fin du traitement, une solution à 2,5 % de bleu Evans dans le sérum physiologique a été injectée par la voie intraveineuse (1 mg/Kg). Immédiatement après, la veine saphène de la patte arrière droite a été stimulée (15v, 2Hz, 1mS) pendant 15 minutes.

L'inflammation neurogénique induite par la stimulation électrique de la veine saphène a été appréciée par la quantité de Bleu Evans extravasé. L'oedème a été également évalué par la différence de poids des échantillons cutanés de la patte arrière gauche (non stimulée) et droite (stimulée).
Les résultats obtenus sont rassemblés dans le tableau 4 ci-après. Le pourcentage de variation est calculé en relation avec le groupe témoin qui ne reçoit que de l'eau bidistillée.

**Tableau 4**

| TRAITEMENT | Bleu Evans extravasé | | OEDEME |
|---|---|---|---|
| | (µg/site) | | (mg) |
| | Med. | 8.13 | 46.50 |
| Eau bidistillée | Mini. | 4.61 | -3.00 |
| | Maxi. | 16.68 | 69.40 |
| | N | 8 8 | |
| | Med. | 1.57 | 5.20 |
| | Mini. | -0.19 | -38.10 |
| SPANTIDE II | Maxi. | 2.97 | 27.80 |
| 30 nmoles | N | 8 | 8 |
| | p | ** | * |
| | % | -81 | -89 |
| | Med. | 4.77 | 25:45 |
| | Mini. | 0.80 | -5.80 |
| Eau de mer | Maxi. | 7.97 | 49.60 |
| + arginine | N | 8 8 | |
| | P | * | NS |
| | % | -41 | -45 |

| | | | |
|---|---|---|---|
| NS : P>0,05 | | | |

### c) Conclusion

En conclusion, l'eau de mer enrichie en arginine provoque une inhibition de l'inflammation neurogénique induite par la stimulation électrique de la veine saphène. Cet effet est statistiquement significatif et représente une diminution de 41 % de l'extravasement cutané du Bleu Evans.
La composition selon l'invention amène également un effet important sur l'oedème.

Il est possible d'ajouter aux compositions selon l'invention un ou plusieurs principes actifs supplémentaires qui renforcent l'efficacité des compositions précédemment décrites. On pourra ainsi adjoindre un agent antibactérien comme la povidone iodée ou un sel de chlorhexidine ou d'hexamidine, un agent antiparasitaire comme le niclosamide, la pelletierine, la quinacrine, le chlorure de pyrvinium ou le chlorure d'embonium, un agent antifongique comme le cyclopirox sel d'olamine, le cotrimazole ou le fenticonazole, un antiprurigineux comme le camphre, le menthol, le phénol ou le salicylate de sodium ou le carbonate de bismuth, des agents antiradicaux libres comme l'acide ascorbique, l'ascorbate de sodium, le tocophérol ou la N-acétylcystéine, des anesthésiques comme la butacaïne, la stovaïne, la novocaïne ou la marcaïne, des agents anti-viraux comme la iododésoxyuridine, la lamivudine, l'acyclovir ou la didésoxyadenosine, des agents antipelliculaires comme la pyrithione zincique ou l'omadine zincique, des produits antiacnéiques comme l'acide caroténoïque, l'acide rétinoïque, le rétinaldéhyde ou le péroxyde de benzoyle, des agents antiséborrhéiques comme le résorcinol, des produits cicatrisants comme le dextranomère ou l'acide hyaluronique, des vitamines du groupe de la Vitamine B (Vitamine B₁, Vitamine B₂, Vitamine B₆, Vitamine PP, Vitamine B₁₂), du groupe de la Vitamine A, du groupe de la Vitamine E et les substances du groupe de la Vitamine D dépourvues d'effet antirachitique.

On entend par agent cicatrisant du groupe des hydrocolloïdes toute substance minérale ou organique susceptible de former un gel au contact de la peau ou des muqueuses et apte à incorporer la préparation selon l'invention.

Les compositions selon l'invention sont destinées à la voie orale notamment sous forme de comprimés ou de gélules après adsorption sur un support inerte, à la voie gingivale sous forme de dentifrice ou d'eau dentifrice, à la voie vaginale, à la voie ophtalmique sous forme de collyres, à la voie auriculaire sous forme de gouttes auriculaires.

L'eau de mer peut assurément être utilisée seule. L'addition d'un aminoacide basique, et notamment de l'arginine, renforce sensiblement les effets de l'eau de mer.
L'eau de mer en effet agit sur les mastocytes et sur les basophiles pour inhiber leur dégranulation. Elle inhibe les effets du VIP, du CGRP, de la Bradykinine.
En outre, l'eau de mer seule inhibe la production de PGE₂ (prostaglandine E₂) sécrétée par des kératinocytes humains.
Les exemples de formulation suivants illustrent l'invention, ils ne la limitent en aucune façon.

### EXEMPLE I

### Collyre

- Eau de mer purifiée, stérilisée, désodée et isotonique 90 %
- Arginine 2%
- Sulfate de dextranne 1 %
- Eau distillée, conservateur qsp

### EXEMPLE II

### Emulsion adoucissante pour peaux sensibles

- Eau de mer purifiée, stérilisée, désodée et isotonique 91 %
- Arginine 2 %
- Emulsion cosmétique pour peaux sensibles q.s.p. (alcools gras, alcools gras polyoxyéthylénés, huile minérale, palmitate d'isopropyle, glycérine, gélifiant, conservateurs, parfums, eau)

### EXEMPLE III

### Composition pour la voie orale

- Eau de mer purifiée, stérilisée 450 g
- Chlorhydrate de lysine 14 g
- Hydroxyéthylcellulose 7 g
- Carbonate de calcium 36 g
- Silicate de magnésium 5 g
- Bentonite 40 g

On adsorbe l'eau de mer purifiée sur le mélange bentonite + hydroxyéthylcellulose pour obtenir une masse pulvérulente que l'on granule puis broye. On ajoute alors le chlorhydrate de lysine puis le carbonate de calcium et finalement le silicate de magnésium
La masse totale est finalement comprimée en 1000 comprimés d'un poids moyeu de 0,520 g.

### EXEMPLE IV

### Composition pour la voie orale

- Eau de mer purifiée, stérilisée 173 g
- Pidolate d'arginine commercialisée 60 g sous la marque Argidone ® (Société PCIB)
- Polyvinylpyrolidone (Kollidon K90) 17 g
- Polyéthylène glycol 4000 120 g
- Carbonate de calcium 120 g
- Talc 10 g

L'eau de mer et le pidolate d'arginine sont adsorbés sur le polyéthylèneglycol 4000. Le mélange pateux en résultant est dilué avec la polyvinylpyrolidone, puis avec le carbonate de calcium. La poudre ainsi obtenue est additionnée du Talc et comprimée en comprimés d'un poids moyen de 0,500 g.

## Revendications

1. Composition pharmaceutique, hygiénique et/ou cosmétique **caractérisée en ce qu'**elle contient de l'eau de mer en quantité comprise entre 30% et 99% du poids total et un aminoacide basique ou l'un de ses sels ou esters, en quantité comprise entre 0,0001% et 10% du poids total ou un extrait végétal et/ou animal, ou du phytoplancton en contenant, en association ou en mélange avec un véhicule ou un excipient inerte, non toxique, approprié pour l'application envisagée.

2. Composition pharmaceutique, hygiénique et/ou cosmétique selon la revendication 1, **caractérisée en ce que** l'aminoacide basique porte un substituant guanidine, amine, amine substitué, un amnonium quaternaire, un méthyle, un groupe carboxamide, un groupe cyano, un groupe phosphonique ou un groupe hydrazide.

3. Composition pharmaceutique, hygiénique et/ou cosmétique selon la revendication 1 ou la revendication 2, **caractérisée en ce que** l'extrait végétal et/ou animal contenant l'aminoacide basique est un extrait d'algue, un extrait de boue marine, thermale et/ou lacustre, un extrait de bactérie, un extrait de plancton.

4. Composition pharmaceutique, hygiénique et/ou cosmétique selon l'une quelconque des revendications 1 à 3, **caractérisée en ce que** l'aminoacide basique est sous forme de l'un de ses sels ou esters tels que le mono- ou le dichlorhydrate, le mono- ou le dibromhydrate, le sulfate, le glutamate, le pidolate, l'ester méthylique ou l'ester éthylique.

5. Composition pharmaceutique, hygiénique et/ou cosmétique selon la revendications 1, **caractérisée en ce que** l'aminoacide basique est l'arginine.

6. Composition pharmaceutique, hygiénique et/ou cosmétique selon la revendication 5, dans laquelle l'arginine est sous forme de pidolate.

7. Composition pharmaceutique, hygiénique et/ou cosmétique selon l'une quelconque des revendications 1 à 4, **caractérisée en ce que** l'eau de mer est prélevée dans les mers, dans les océans ou dans les infiltrations ou les résurgences, qu'elle est filtrée et qu'elle est stérilisée par filtration stérilisante complémentaire.

8. Composition pharmaceutique, hygiénique et/ou cosmétique selon la revendication 7, **caractérisée en ce que** l'eau de mer, filtrée et stérilisée, est amenée à l'isotonie par dilution ou par désionisation.

9. Composition pharmaceutique, hygiénique et/ou cosmétique selon la revendication 1, **caractérisée en ce que** la quantité d'eau de mer représente de 60% à 95% du poids total de la composition.

10. Composition pharmaceutique, hygiénique ét/ou cosmétique selon la revendication 1, **caractérisée en ce que** la quantité d'aminoacide basique représente de 0,0005% à 2% du poids total de la composition.

11. Composition pharmaceutique, hygiénique et/ou cosmétique selon l'une quelconque des revendications 1 à 10, **caractérisée en ce qu'**elle contient en outre un agent choisi parmi les agents antibactériens, les antiparasitaires, les antifongiques, les antiprurigineux, les anti-radicaux libres, les anesthésiques, les agents antiviraux, les agents antipelliculaires, les produits anti-acnéiques, les agents antiséborrhéiques, les produits cicatrisants sous forme d'hydrocolloïdes et les agents vitaminiques.

12. Composition pharmaceutique, hygiénique et/ou cosmétique selon l'une quelconque des revendications 1 à 11, **caractérisée en ce qu'**elle renferme en outre un agent de régulation du pH.

13. Composition pharmaceutique, hygiénique et/ou cosmétique selon l'une quelconque des revendications 1 à 12, **caractérisée en ce que** le pH est réglé dans une zone s'étendant de 5,5 à 9.

14. Composition pharmaceutique, hygiénique et/ou cosmétique selon l'une des revendications 1 à 12 à laquelle on associe un aminoglycanne, un polysaccharide ou un polymère polysaccharidique.

15. Compositions pharmaceutiques, hygiéniques et/ou cosmétiques selon l'une des revendications 1 à 14 **caractérisée en ce qu'**elles se présentent sous l'une des formes appropriées pour l'administration par voie orale, par voie topique, par voie gingivale, par voie vaginale, par voie auriculaire et/ou par voie ophtalmique.

16. Utilisation d'une composition selon l'une quelconque des revendications 1 à 15, pour la réalisation d'un médicament apte à prévenir et/ou traiter les affections liées à une libération de médiateurs de l'inflammation et/ou de l'allergie tels que l'histamine ou les cytokines.

17. Utilisation d'une composition selon la revendication 16, pour réaliser un médicament apte à prévenir et/ou traiter les manifestations allergiques et/ou inflammatoires, notamment au niveau de la peau, des yeux, des bronches et du nez.

## Claims

1. A pharmaceutical hygienic, and/or cosmetic composition wherein it contains sea water, in an amount ranging from 30 to 99 % of the total weight and a basic aminoacid or, one of its salts or esters in an amount ranging between 0.0001 and 10 % or the total weight, or a vegetal and/or animal extract or phytoplankton containing it, associated or admixed with an inert nontoxic vehicle or excipient suitable for the contemplated use.

2. A pharmaceutical, hygienic and/or cosmetic composition according to claim 1, wherein the basic aminoacid bears a guanidino, an amino, a substituted amino, quaternary ammonium methyl, carboxamido group, a cyano group, a phosphono group or a hydrazido group as the substituent.

3. A pharmaceutical hygienic and/or cosmetic composition according to claim 1 or claim 2, wherein the vegetal and/or animal extract containing the basic amino acid is am alga extracts, an extract of sea, thermal and/or lake mud, an extract of bacteria or an extract of plankton.

4. A pharmaceutical, hygienic and/or cosmetic compositian according to any of claims 1 to 3, wherein the basic amino acid is in the form of one of its salts or esters such as the mono-or dihydrochloride, the mono or dihydrobromide, the sulphate, the glutamate, the pidolate, the methyl ester or the ethylester

5. A pharmaceutical hygienic and/or cosmetic composition according to claim 1, wherein the basic aminoacid is arginine

6. A pharmaceutical hygienic and/or cosmetic composition according to claim 5 wherein arginine is in the form of a pidolate.

7. A pharmaceutical hygienic and/or cosmetic composition according to any of the claims 1 to 4, wherein the sea water is taken from the seas, from the oceans, in the soil leakages or reappearances, filtered and sterilized by means of a complementary sterilizing filtration.

8. A pharmaceutical hygienic and/or cosmetic composition according to claim 7, wherein the filtered and sterilized sea water, is brought to isotony by dilution or desionization.

9. A pharmaceutical hygienic and/or consmetic composition according to the preceding claims wherein the amount of sea water represents from 80 to 95 % of the total weight of this composition.

10. A pharmaceutical hygienic and/or cosmetic composition according to claim 1 wherein the amount of basic aminoacid represents from 0,0005 % to 2 % of the total weight of the composition.

11. A pharmaceutical hygienic and/or cosmetic composition according to any of the claims 1 to 10 wherein it morcover contains an agent selected among the antibacterial agents, the antiparasitive agents, the anti fungi, the antipruriginous, the free radicals scavengers, the anesthetics, the antiviral agents, the anti-dandruff agents, the anti-acneic agents, the anti seborrheic agents, the cicatrising products in the form of hydrocolloids and the vitaminie agents.

12. A pharmaceutical hygienic and/or cosmetic composition according to any of claims 1 to 10 wherein it contains further an agent which regulates the pH.

13. A pharmaceutical hygienic and/or cosmetic composition according to any of claims 1 to 10 wherein the pH is regulated within an area extending from 5.5 to 9.

14. A pharmaceutical hygienic and/or cosmetic composition according to any of claims 1 to 12, to which an aminoglycan, a polysaccharide or a polysaccharidic polymer is admixed.

15. A pharmaceutical hygienic and/or cosmetic compositions according to any of claims 1 to 14 wherein they are presented in one of the forms suitable for the administration by oral way, topical way, gingival way, vaginal way, uricular way and/or ophtalmological way.

16. Use of a composition according to any of claims 1 to 15, for the production of a drug that is able to prevent and/or treat the disorders link to a release of mediators of inflammation or allergy such as histamine or cytokins.

17. Use of a composition in accordance with one of claims 1 to 16, intended for the production of a medicine which is able to treat or prevent the inflammatory phenomena, namely at the level of the skin, of the eyes, of the bronchial tract and the nose.

## Patentansprüche

1. Pharmazeutische, hygienische und/oder kosmetische Zusammensetzung, **dadurch gekennzeichnet, dass** sie Meerwasser in einer Menge zwischen 30% und 99% des Gesamtgewichts und eine basische Aminosäure oder eines ihrer Salze oder einen ihrer Ester oder einen pflanzlichen und/oder tierischen Extrakt oder Phytoplankton, welche diese enthalten, in einer Menge zwischen 0,0001% und 10% des Gesamtgewichts in Assoziation oder Mischung mit einem inenen, nicht toxischen, für die beabsichtigte Anwendung geeigneten Vehikel oder Exzipiens enthalten.

2. Pharmazeutische, hygienische und/oder kosmetische Zusammensetzung nach Anspruch 1, **dadurch gekennzeichnet, dass** die basische Aminosäure einen der folgenden Substituenten trägt: Guanidin, Amin, substituiertes Amin, ein quaternäres Ammonium, ein Methyl, eine Carboxamidgruppe, eine Cyanogruppe, eine Phosphonsäuregruppe oder eine Hydrazidgruppe,

3. Pharmazeutische, hygienische und/oder kosmetische Zusammensetzung nach Anspruch 1 oder Anspruch 2, **dadurch gekennzeichnet, dass** der die basische Aminosäure enthaltende pflanzliche und/oder tierische Extrakt ein Algenextrakt, ein Extrakt aus Meeres-, Thermal- und/oder Süßwasserschlamm, ein Bakterienextrakt, ein Planktonextrakt ist.

4. Pharmazeutische, hygienische und/oder kosmetische Zusammensetzung nach einem der Ansprüche 1 bis 3, **dadurch gekennzeichnet, dass** die basische Aminosäure in Form von einem ihrer Salze oder Ester wie dem Mono- oder dem Dichlorhydrat, dem Mono- oder dem Dibromhydrat, dem Sulfat, dem Glutamat, dem Pidolat, dem Methylester oder dem Ethylester vorliegt.

5. Pharmazeutische, hygienische und/oder kosmetische Zusammensetzung nach Anspruch 1, **dadurch gekennzeichnet, dass** die basische Aminosäure Arginin ist.

6. Pharmazeutische, hygienische und/oder kosmetische Zusammensetzung nach Anspruch 5, in der Arg in in in Form von Pidolat vorliegt.

7. Pharmazeutische, hygienische und/oder kosmetische Zusammensetzung nach einem der Ansprüche 1 bis 4, **dadurch gekennzeichnet, dass** das Meerwasser den Meeren, den Ozeanen oder Versickerungen oder wieder zutage getretenen Versickerungen entnommen wird, dass es filtriert wird und dass es durch zusätzliche Sterilfiltration sterilisiert wird.

8. Pharmazeutische, hygienische und/oder kosmetische Zusammensetzung nach Anspruch 7, **dadurch gekennzeichnet, dass** das fltrierte und sterilisierte Meerwasser durch Verdünnung oder durch Entionisierung isotonisiert wird.

9. Pharmazeutische, hygienische und/oder kosmetische Zusammensetzung nach Anspruch 1, **dadurch gekennzeichnet, dass** die Menge an Meerwasser 60% bis 95% des Gesamtgewichts der Zusammensetzung ausmacht.

10. Pharmazeutische, hygienische und/oder kosmetische Zusammensetzung nach Anspruch 1 , **dadurch gekennzeichnet, dass** die Menge an basischer Aminosäure 0,0005% bis 2% des Gesamtgewichts der Zusammensetzung ausmacht.

11. Pharmazeutische, hygienische und/oder kosmetische Zusammensetzung nach einem der Ansprüche 1 bis 10, **dadurch gekennzeichnet, dass** sie außerdem ein Mittel, gewählt unter den antibakteriellen Mitteln, den Antiparasitika, den Antimykotika, den Antipruriginosa, den Mitteln gegen freie Radikale, den Anästhetika, den Antivirusmitteln, den Antischuppenmitteln, den Antiakne-Produkten, den Antiseborrhoika, den Wundheilungsprodukten in Form von Hydrokolloiden und den Vitaminmitteln, enthält.

12. Pharmazeutische, hygienische und/oder kosmetische Zusammensetzung nach einem der Ansprüche 1 bis10 , **dadurch gekennzeichnet, dass** sie außerdem ein Mittel zur Einstellung des pH-Werts enthält.

13. Pharmazeutische, hygienische und/oder kosmetische Zusammensetzung nach einem der Ansprüche 1 bis 12, **dadurch gekennzeichnet, dass** der pH in einem Bereich eingestellt wird, der sich von 5,5 bis 9 erstreckt.

14. Pharmazeutische, hygienische und/oder kosmetische Zusammensetzung nach einem der Ansprüche 1 bis 12, die mit einem Aminoglykan, einem Polysaccharid oder einem Polysaccharid-Polymer assoziiert wird.

15. Pharmazeutische, hygienische und/oder kosmetische Zusammensetzung nach einem der Ansprüche 1 bis 14, **dadurch gekennzeichnet, dass** sie in einer der Formen vorliegt, die sich zur Verabreichung auf oralem Wege, auf topischem Wege, auf gingivalem Wege, auf vaginalem Wege, auf aurikulärem Wege und/oder auf ophtrtaimischem Wege eignen.

16. Verwendung einer Zusammensetzung nach einem der Ansprüche 1 bis 15 zur Herstellung eines Medikaments, das dazu geeignet ist, Erkrankungen zu verhüten und/oder zu behandeln, die mit einer Freisetzung von Entzündungs- und/oder Allergievermittlem wie Histamin oder den Zytokinen verbunden sind.

17. Verwendung einer Zusammensetzung nach einem der Ansprüche 1 bis 16 zur Herstellung eines Medikaments, das dazu geeignet ist, allergische und/oder entzündliche Erscheinungen, insbesondere auf der Ebene der Haut, der Augen, der Bronchien und der Nase, zu verhüten und/oder zu behandeln.
